# EUROPEAN PATENT APPLICATION

(11) **EP 2 149 581 A1**
(43) Date of publication of application: **03.02.2010**
(21) Application number: 07834945.3
(22) Date of filing: 20.04.2007
(51) Int. Cl.: C07H 15/10, C08L 3/00, C08J 3/00, C12Q 1/68, C12N 11/00, G01N 33/531

(54) **MONOMER AND COMPOSITION FOR PRODUCING LOW-PERCENTAGE HYDROGEL AND/OR HYDROGEL HAVING A LOW CROSS LINKAGE CONTENT, A HYDROGEL AND A BIOCHIP BASED THEREON**

(71) Applicant: Institut Molekulyarnoi Biologii Im. V.A. Engelgardta Rossiiskoi Akademii Nauk, 119991, Moscow (RU)
(72) Inventor: PANKOV, Sergei Vasilievich, Moscow, 117152 (RU); RUBINA, Alla Yurievna, Moscow, 117313 (RU); SOMOVA, Olga Georgievna, Moscow, 111141 (RU); MOISEEEVA, Olga Vladimirovna, Moscow, 111141 (RU); CHECHETKIN, Vladimir Romanovich, Moskovskaya obl., 142190 (RU); SOROKIN, Nikolai Vladimirovich, Moscow, 111141 (RU); DONNIKOV, Maxim Yurievich, Rostovskaya obl., 346000 (RU); BUTVILOVSKAYA, Veronika Igorevna, Stavropolsky krai, 357101 (RU); KONOVALOVA, Elizaveta Vladimirovna, Moskovskaya obl., 141070 (RU); ZASEDATELEV, Alexandr Sergeevich, Moscow, 117418 (RU)
(74) Representative: HOFFMANN EITLE
(86) International application number: PCT/RU2007/000196
(87) International publication number: WO 2008/130263

(57) **Abstract**

The invention relates to molecular biology and bioorganic chemistry, in particular to hydrogels based on cross-linked polymers with oligonucleotides, proteins, nucleic acids or any other biologically significant compounds which are immobilized therein, and to biochips which are based on the inventive hydrogels and are used in molecular biology for DNA sequencing and mapping, for detecting mutations, in analytical chemistry, for immunoassay and in many other medical applications.

## Description

### Technical Field

The invention relates to molecular biology, bioorganic chemistry and is concerned with hydrogels on cross-linked polymers basis with oligonucleotides, proteins, nucleic acids or any other biologically significant compounds which are immobilized therein, and to biochips which are based on the inventive hydrogels and are used in molecular biology for DNA sequencing and mapping, for detecting mutations, in analytical chemistry, for immunoassay and in many other medical applications.

### State of the Art

Known in the art are hydrogels with biologically active compounds based on cross-linked polymers being prepared by a method of radical-initiated polymerization of acrylamide, methacrylamide, N-[tris(hydroxymethyl)methyl], acrylamide, -2-hydroxyethylmethacrylate in the presence of modified or unmodified biologically significant compounds and various "crosslinking" agents, such as: N, N'-methylenebisacrylamide, N,N'-(1,2-dihydroxyethylenebisacrylamide), polyethylene glycol diacrylate (PEGDA) and N,N'-diallyl-substituted diamide of tartaric acid (Patents US 5932711, RU 2175972, US 6656725, RU 2206575, RU 2216547).

As known, atmospheric oxygen inhibits the polymerization of the above-listed monomers and for producing hydrogels having a sufficient mechanical strength, in air polymerization, it is necessary to increase the total content of a monomer and a cross-linker up to 10% and more, as is the case with US patent 5932711. Under the given conditions, the immobilization state of a biologically significant compound in immobilization in a drop of 1-2 nl in volume does not exceed 10%.

The problem of inhibiting the polymerization of the above-listed monomers and "cross-linkers" with atmospheric air is eliminated in the polymerization in an inert gas atmosphere (nitrogen, argon, carbon dioxide, to mention only few). Under such conditions there can be produced gels having the total content of a monomer and a cross-linker ≥ 3%, a portion of the cross-linker can be reduced up to 3% (Patents RU 2206575, RU 2216547), and the immobilization state in said 1-2nl drop can be increased up to 50-60%.

However, to solve some tasks dealing with hybridization on biochip gel elements, of large DNA fragments, for increasing a polymerase chain reaction yield, given PCR carried out on the biochip gel elements, and for enhancing the sensitivity of a quantitative immunoassay on the biochip gel elements, it is necessary to produce hydrogels whose porosity is higher than that of known analogues. Said porosity can be achieved by considerably diminishing in a composition for producing gels, the content of a "cross-linker" or a monomer and the "cross-linker" simultaneously. Polymerization of such compositions cannot be accomplished using the known compositions (Patents US 5932711, RU 2175972, US 6656725, RU 2206575, RU 2216547) and may call for special methods of approach.

The task set is tackled in the invention, as being claimed and as set forth in the application.

### Disclosure of the Invention

As a result of comprehensive research, the authors of the present invention have suddenly discovered that use can be made of, as a monomer for producing low-percentage hydrogels and "cross-linkage" low-content hydrogels, an O-substituted sugar and/or N-substituted aminosugar-based derivative comprising an unsaturated residue, given a hydroxyl group or an amino group, respectively.

The present invention claims low-percentage hydrogels and "cross-linkage" low-content hydrogels with oligonucleotides, proteins, nucleic acids or other biologically significant compounds immobilized therein, which are produced in the chemical or actinic radical initiated polymerization of compositions having a low content of a cross-linker or a monomer and the cross-linker simultaneously.

Hydrogels featuring new properties can be obtained in the chemical or photochemical initiation of the polymerization of compositions with new monomers based on N-substituted aminosugars and O-substituted sugars using the known methods of biochips production (Patents RU 2206575, RU 2216547).

Thus, in its first aspect, the present invention relates to a monomer for the production of a low-percentage hydrogel and/or hydrogel having a low content of "cross-linkage" that is a derivative based on an O-substituted sugar and/or N-substituted aminosugar comprising unsaturated residue, given a hydroxyl group or an amino group, respectively.

In one of the alternative embodiments, the sugar represents a low-molecular compound of a class of carbohydrates, selected from the group consisting of monosaccharides and oligosaccharides. In a preferable embodiment, the sugar is a compound selected from the group consisting of sucrose, D-glucose, D-allose, D-altrose, D-mannose, D-gulose, D-idose, D-talose, D-fructose, D-ribose, D-arabinose, D-xylose, D-lyxose, D-eritrulose, D-ribulose, D-xylulose, D-Psicose, D-sorbose, D-magatose or their desoxyanalogue or their analogue with the L-configuration or a mixture of isomers with the L and D-configurations.

In another embodiment, the aminosugar represents a natural or synthetic sugar in which one or several hydroxyl groups are substituted with an amino group. In a preferable embodiment, the aminosugar represents a compound selected from the group consisting of 2-amino-2-desoxy-D-glucose, 2-amino-2-desoxy-D-allose, 2-amino-2-desoxy-D-altrose, 2-amino-2-desoxy-D-mannose, 2-amino-2-desoxy-D-gulose, 2-amino-2-desoxy-D-idose, 2-amino-2-desoxy-D-talose, 2-amino-2-desoxy-D-galactose or an analogue thereof with the L-configuration or a mixture of isomers with the D and L-configurations.

In a further embodiment, the monomer comprises as unsaturated residue, a group having a multiple bond capable of causing the reaction of photo-or chemical initiated polymerization. In a preferable embodiment, the unsaturated residue used is represented by a methacryl, acryl, vinyl, allyl, 4-vinylbenzoyl or methylene-4-phenylenevinyl group.

In another aspect thereof, the invention relates to a composition (C) for producing a low-percentage hydrogel and/or hydrogel with a low "cross-linkage" content, comprising:
M - monomer of the present invention forming a base of a formable gel;
C - cross-linker;
S - solvent.

In one of the embodiments, the additional usable monomer (M) is represented by at least one other unsaturated monomer. In a preferable embodiment, the at least one other unsaturated monomer used is represented by a compound selected from the group being comprised of methacrylamide, acrylamide, 2-hydroxyethylmethercrylate and 2-hydroxyethylmethacrylamide.

In another embodiment, the cross-linker (C) used is represented by at least one unsaturated compound comprising two or more multiple bonds. In a preferable embodiment, the at least one unsaturated compound used is represented by a compound selected from the group comprising N, N'-methylenebisacrylamide, N,N'- (1,2-dihydroxyethylene)bisacrylamide, polyethylene glycol diacrylate.

In a further embodiment, a solvent (S) is a solution of an organic and/or inorganic compound. In a preferable embodiment, the solvent is selected from the group consisting of an aqueous solution of glycerol, sugar, betaine, polyalcohol; solution of glycerol, sugar, betaine or polyalcohol in dimethylsulfoxide (DMSO); solution of glycerol, sugar, betaine or polyalcohol in dimethylformamide (DMFA).

In a still further embodiment, the composition of the present invention further comprises at least one initiator and/or promoter of polymerization (Init). In a preferable embodiment, the initiator and/or promoter of polymerization is an initiator for chemical initiated polymerization and/or promoter for phoroinitiated polymerization. In the most preferable embodiment at least one initiator and/or promoter of polymerization is selected from the group consisting of ammonium persulfate, potassium persulfate, hydrogen peroxide, benzoyl peroxide, azoisobutyronitrile (AIBN), methylene blue, fluoroscein, N, N, N', N'-tetramethylethylenediamine, 4-(N,N-dimethylamino)pyridine, triethylamine, and acetone.

In a yet another embodiment, the composition of the present invention further comprises at least one inhibitor of polymerization (Inh). In a preferable embodiment at least one inhibitor of polymerization (Inh) represents a compound precluding spontaneous polymerization. In the most preferable embodiment at least one compound precluding the spontaneous polymerization is a compound selected from the group consisting of n-benzoquinone, o-nitrophenol, p-nitrophenol, nitrobenzene, o-dinitrobenzene, m-dinitrobenzene and p-dinitrobenzene.

In the following embodiment, the composition of the present invention further comprises at least one fluorescent stain (Sf). In a preferable embodiment at least one stain used is represented by the stain selected from the group consisting of Texas Red, fluorescein, Cy 5, Cy 3, BODIPY.

In one more embodiment of the present invention, the composition further comprises a biologically significant compound (B). In a preferable embodiment, the biologically significant compound (B) is capable of being immobilized in a hydrogel at the moment of its formation. In the most preferable embodiment, the composition of the present invention comprises, as a biologically significant compound, at least one compound, selected from the group consisting of an oligonucleotide, a nucleic acid, a protein, an oligosaccharide, an antibody or a low - molecular ligand.

In another aspect thereof the present invention relates to a hydrogel representing a low-percentage hydrogel and/or hydrogel having a low "cross-linkage" content that is produced by the photo- or chemical induced polymerization of the composition of the present invention.

In a further aspect thereof the present invention relates to a biochip representing a substrate with a hydrogel layer immobilized thereon in accordance with the present invention.

In one of the embodiments the biochip is characterized by the fact that a hydrogel layer is continuous.

In an alternative embodiment, the biochip is **characterized in that** a hydrogel layer is divided by voids into cells. In a preferable embodiment, the cells form a regular unidimensional or two-dimensional structure (array).

In another embodiment, the biochip is characterized by the fact that a gel layer comprises at least one immobilized biologically significant compound. In one of the preferable embodiments, the immobilization of at least one biologically significant compound in the hydrogel layer is effected at the moment of hydrogel formation in the chemical- and photochemical initiated polymerization. In another preferable embodiment, the immobilization of at least one biologically significant compound in a hydrogel layer is effected after the hydrogel has been formed.

In still another embodiment, the biochip is characterized by the fact that each and every hydrogel cell of the biochip comprises an immobilized fluorescent stain.

### Brief List of Figures

The invention will now be described in greater detail with a reference made to Figures, in which:
Fig. 1 shows the appearance of biochip gel elements (A, B) which are produced by a method of the photoinitiated polymerization of compositions K1-K74 (cf. Table). Biochips (A, B) have been produced according to Example 2 (method 1). Photographs have been obtained in visible transmitted light.
Fig. 2 shows the result of hybridization of a fluorescent labeled probe (126 n.o.) on an oligonucleotide biochip produced with the use of compositions K85, K86.
Oligonucleotides 5'-TTC TGG TCC ATG AAT TGG-OCH₂CH(CH₂OH)(CH₂)₄-NH₂(**A**) and 5'-GTT CTG GTC TAT GAA TTG G-OCH₂CH(CH₂OH)(CH₂)₄-NH₂(**B**) were immobilized in hydrogels produced on K85, K86 compositions basis, said compositions being polymerized according to Example 2 (method 1). The biochips so obtained were hybridized for 3 hours to a fluorescent labeled probe (126 n.o.) that is fully complementary to the oligonucleotide (A).

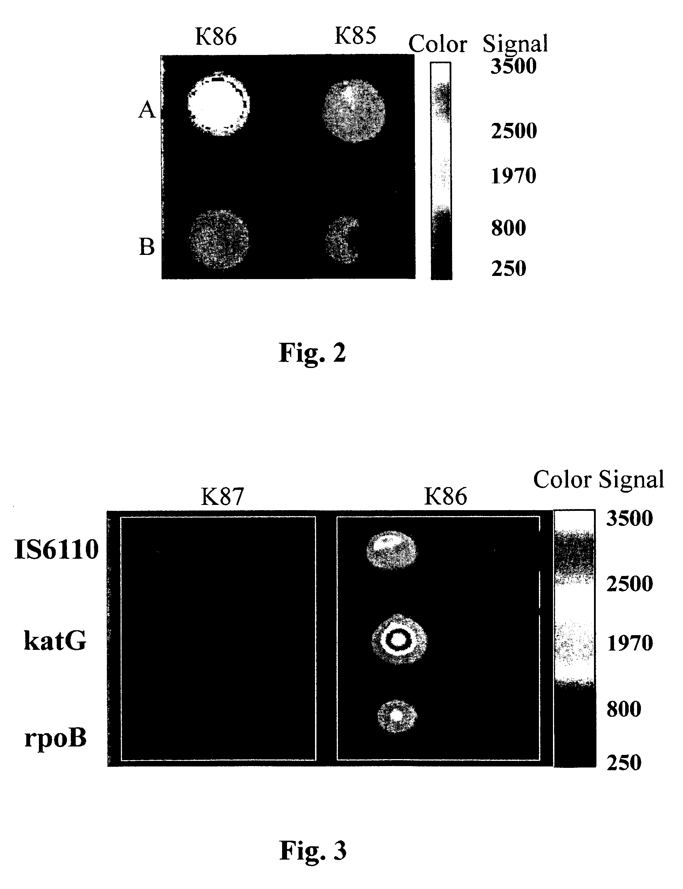

Fig. 3 illustrates a comparative efficacy of an asymmetric multiplex PCR proceeding on biochip gel elements. The gel elements are produced on the basis of one composition (K87) out of those known in literature (patent RU 2216547) and one of the compositions, as called for in the claims (K86) (cf. Table 1). The efficacy of the PCR is testified by a value of a fluorescent signal entered from a biochip element after the reaction.

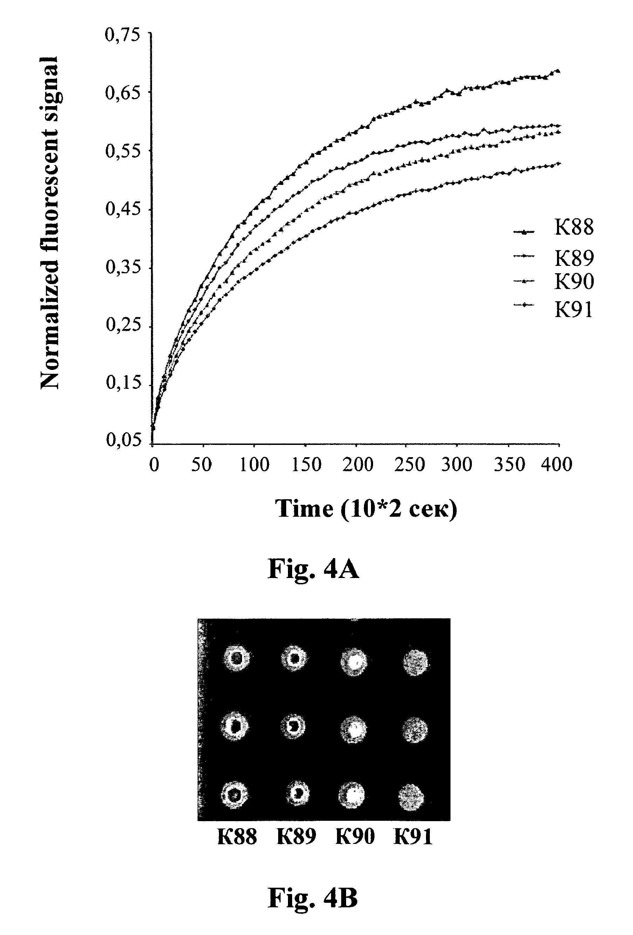

Fig. 4(A) reflects a kind of kinetic curves (growth of a fluorescent signal in time) in gels of varying compositions for the formation of a binary antibody-antigen complex. The antigen selected is represented by a serological prostate anti-cancer oncomarker (PSA) labeled by a fluorescent Cy5 stain. In the gels based on compositions K88-K91 are immobilized monoclonal antibodies to said marker in equal concentrations. In all gels, the basic monomer used was represented by 2-methacrylamido-2-desoxy-D-glucose.
Fig. 4(B) demonstrates the fluorescent image of a biochip on completion of an assay.

### Execution of the Invention

The present invention offers monomers based on O-substituted sugars and N-substituted aminosugars to produce hydrogels by a method of photo-or chemical initiated polymerization. The nature of monomers is such that it allows to produce low-percentage hydrogels and gels having a low "cross-linkage" content, using the known methods of producing biochips (Patents RU 2206575, RU 2216547).

The unsaturated group of a monomer is a methacryl, acryl, vinyl, allyl, 4-vinylbenzoyl, methylene-4-phenylenevinyl or any other one reactive with the photo- or chemical initiated polymerization and being on an O atom in sugars and on an N atom in aminosugars.

Sugars represent the low-molecular compounds of a class of carbohydrates, concretely - monosaccharides and oligosaccharides (A. Terney "Sovremennaya organicheskaya khimiya". V. 2, M.: MIR Publishers, 1981, pp. 420, 453) such as: sucrose, D-glucose, D-allose, D-altrose, D-mannose, D-gulose, D-idose, D-talose, D-fructose, D-robose, D-arabinose, D-xylose, D-lyxose, D-erithrulose, D-ribulose, D-xylulose, D-psicose, D-sorbose, D-magatose or their desoxyanalogues or analogues with the L-configuration or a mixture of isomers with L and D-configurations.

Aminosugars represent natural or synthetic sugars in which one or a plurality of hydroxyl groups are substituted with an amino group, for example: 2-amino-2-desoxy-D-glucose, 2-amino-2-desoxy-D-allose, 2-amino-2-desoxy-D-altrose, 2-amino-2-desoxy-D-mannose, 2-amino-2-desoxy-D-gulose, 2-amino-2-desoxy-D-idose, 2-amino-2-desoxy-D-talose, 2-amino-2-desoxy-D-galactose or their analogues with the L-configuration or a mixture of isomers with D and L-configurations.

O- and N-substituted sugars and aminosugars are produced by acetylation or alkylation of the sugars and aminosugars with various derivatives of unsaturated carboxylic acids or unsaturated alcohols or alkylhalides, respectively.

Monomers based on O-substituted sugars and N-substituted aminosugars are used for producing low-percentae hydrogels or gels having a low "cross-linkage" content.

Hydrogels are produced in the photo- or chemical initiation of the polymerization of compositions (C=M+C+S) including a monomer (M), a cross-linker (C), and a solvent (S). The compositions may further include a biologically significant compound (B), an initiator or a polymerization promoter (Init), a polymerization inhibitor (Inh).

Monomer (M) is an O-substituted sugar or an N-substituted aminosugar comprising on an O-atom or an N-atom, any unsaturated group, specifically: methacryl, acryl, vinyl, allyl, 4-vinylbenzoyl and methylene-4-phenylenevinyl one reactive with the photo- or chemical initiated polymerization.

Besides, the monomer used can be represented by the mixtures of O - substituted sugars or N-substituted aminosugars with conventional monomers for the reaction of photo- and chemical initiated polymerization, with the amides or esters of unsaturated acids, in particular: methacrylamide, acrylamide, 2-hydroxyethylmethacrylate, 2- hydroxyethylmethacrylamide, to mention only few.

Employment of various combinations of sugar-containing monomers and sugar-free monomers in a composition permits producing hydrogels with the specified content of sugar residues for one polymer monomer unit.

The product of monomer polymerization is a line structure polymer comprising one sugar molecule for one or several polymer monomer units.

A cross-linker (C) is any unsaturated compound comprising two or more multiple bonds, specifically: N, N'-methylenebisacrylamide, N,N'-(1,2-dihydroxyethylene)bisacrylamide and polyethylene glycol diacrylate. The polymerization product of the cross-linker (C) is a cross-linked polymer with a two-dimensional- or three-dimensional network.

On co-polymerization of a monomer (M) and a cross-linker (C) there are provided cross-linked polymers swollen in aqueous media (hydrogels). Depending on the total (M) and (C) content and their mutual ratio, one can obtain the hydrogels with necessary characteristics as to diffusion, mechanical strength, swelling state, and so on.

While using as (M) monomer an O-substituted sugar and/or N-substituted aminosugar, as well as certain cross-linkers (C), hydrogels can be provided in which O< M+C≤ % (when O ≤ C/(C+M) ≤0% and O < M+C < 50% (when 0 ≤ C/(C+M) ≤ 3%) without changing a method of producing biochips, as referred to in patents RU 2206575, RU 2216547.

A biologically significant compound (B) represents any compound capable of tying, directly or in a mediated manner, molecules of a nucleotide, protein or sugar nature, in particular: an oligonucleotide, a nucleic acid, a protein (antibody, receptor, etc.), an oligosaccharide or a low molecular ligand. Preferably the biologically significant compound (B) is immobilized in a hydrogel at the moment of its formation in photo-or chemical initiated polymerization. For immobilization, the compound (B) should include in its structure:
or any unsaturated group capable of polymerizing with a monomer (M) and a cross-linker (C), particularly: acrylamide or methacrylamide;
or a group chemically active with respect to the monomer (M) and/or the cross-linker (C) and/or intermediates originating in forming a cross-linked polymer, particularly: amino - or sulfhydryl groups. However, a biologically significant compound can be immobilized even after the hydrogel has already been formed.

The initiators or promoters of radical polymerization (Init) are well-known initiators for chemical initiated polymerization and promoters for photo-initiated polymerization, particularly: ammonium persulfate, potassium persulfate, hydrogen peroxide, benzoyl peroxide, azoisobutyronitrile (AIBN), methylene blue, fluorescein, N,N,N',N'-tetramethylethylenediamine, 4-(N,N-dimethylamino)pyridine, triethylamine, and acetone.

Polymerization inhibitors (Inh) are compounds involved in a transfer chain process, with the formation of intermediates incapable of providing chain continuity, particularly: n-benzoquinone, o-nitrophenol, p-nitrophenol, nitrobenzene, o-)m-, p-)-dinitrobenzenes.

The inhibitors within a composition (C) are used for preventing spontaneous polymerization on storage of the compositions (C).

Solvent (S) is a medium for carrying out photo- or chemical initiated polymerization. The solvent can be of an organic or inorganic nature and also represent solutions of organic and inorganic compounds, particularly: aqueous solutions of glycerol, sugars, betaine, polyalcohols; solutions of glycerol, sugars, betaine or polyalcohols in dimethylsulfoxide (DMSO); solutions of glycerol, sugars, betaine or polyalcohols in dimethylformamide (DMFA). Basic requirements imposed on the solutions are as follows: high boiling temperature, relatively low viscosity and ability to maintain a normal course of polymerization.

Fluorescent stain (Sf) is intended for determining a degree of polymerization process proceeding. The fluorescent stain used can be represented by, for example, Texas Red, fluorescein, Cy5, Cy3, BODIPY and other fluorescent stains.

The aforesaid compositions (C) comprising biologically significant compounds are used for producing biological microchips.

A biochip is a substrate-formed gel layer. The gel layer can be divided by voids into cells, each one containing or not containing immobilized biologically significant compounds, and the biologically significant compounds immobilized in various cells can differ as to their nature and properties. The cells form a regular unidimensional or two-dimensional structure (array). The biochip cells not comprising a biologically significant compound can be used for the determination of the non-specific sorption of a sample analyzed.

Biological microchips using a composition (C) comprising O-substituted sugars and/or N-substituted aminosugars can be produced by conventional methods (as disclosed, for example, in patents RU 2206575, RU 2216547) comprising the following steps:
a. producing a composition (C);
b. transfer of the composition (C) onto a substrate;
c. photo- or chemical initiated radical polymerization of the composition (C);
d. "washing-off" of the composition (C) components not entering into the composition of a formable gel;
e. "drying" of biochip gel elements.

A step of producing a composition resides in that all the components of the composition are carefully mixed in specific ratios to obtain a homogeneous solution, degassed, transferred into a container for storage and subsequent application to a substrate, for example, a microtiter plate, and are stored at 4°C.

The step of compositions (C) transfer to a substrate resides in that the compositions are transferred in the form of drops to the substrate by means of any device or arrangement, specifically by an automatic apparatus (robot) provided with one or a plurality of rod-like microdispensers or noncontact jet type microdispensers.

A substrate can be made from any known material, particularly glass, ceramics, metal, and plastic. The surface of the substrate should have either strong adhesive properties or comprise active groups reactive in chain transfer in forming a hydrogel on its surface. The active group can be any group of an organic or inorganic nature, specifically: methacrylic, acrylic, vinyl, allyl, 4-vinylbenzoyl, methylene-4-phenylenevinyl, amino-, sulfo, hydrocarbon groups, and so on.

The step of photo- or chemical radical initiated polymerization resides in that the substrates with thansferred compositions (C) are subjected to UV radiation having a wave length of ≥ 254 in photoinitiation or are exposed to various effects for the chemical initiation of polymerization, particularly: temperature rise, maintenance in a polymerization promoter atmosphere, etc.

Photo- or chemical polymerization initiation is carried out in air or oxygen-free atmosphere, particularly: nitrogen, argon, and carbon dioxide.

The step of a washing-off of composition (C) components not entered in the composition of a hydrogel is that substrates with the compositions (C) after exposure to UV radiation are washed off with buffer solutions and distilled water.

The step of drying biochip gel elements is that substrates with immobilized gel elements are dried in air or in a centrifuge and as such are stored and utilized.

In some cases, steps d) and e) are not obligatory. Thus, oligonucleotide biochips obtainable with the use of only steps a) - c) can successfully be used for hybridizations and PCR. Protein biochips obtainable without steps d) and e) are preserved very well and function after having been stored over an appreciable time.

The invention will further be exemplified by the preferable alternative embodiments of the invention. The illustrative examples shouldn't be taken as limiting an amount of the invention. Persons skilled in the art will find numerous improvements which likewise comprise the scope of claims of the present invention and which are reflected in the claims.

### Examples

### Example 1. 2-methacrylamido-2-desoxy-D-glucose synthesis

Anhydrous methanol (50 ml) is added with triethylamine (1,52 g, 15, 1 mmole) and then with 2-amino-2-desoxy-D-glucose hydrochloride (2,75 g, 9,7 mmole). The suspension thus formed under stirring is added more with an anhydride of methacrylic acid (2,43 g, 15,7 mmole) and further stirred for 12 hours at room temperature. On lapse of time, the solution is filtered and a filtrate is evaporated at T=65°C to a volume of 17 ml and allowed to stay at T=15°C for 12 hours. The precipitate is filtered, washed with acetonitrile (2 x 10 ml) and dried in air at room temperature thereby to protect from direct sun rays. The product represents a white amorphous powder. Yield: 1,86 g (61%); fusing temperature = 205 - 206°C.

**Spectrum H¹ NMR** (D₂O), δ: 1,76 (c, 3H, CH₃), 3,26-3,81 (m, 7H, H², 2H³, H⁴, H⁵, 2H⁶), 5,09 (d, J=3,43 Hz, 1H, H¹), 5, 30 (c, 1H, CH₂=); 5,52 (c, 1H, CH₂=).

Calculated for C16H29NO5: C: 60,93%; H: 9,27%. Found: C: 60,85%; H: 9,32%.

### Example 2. Production of biochips

### Method 1. Production of oligonucleotide biochips

For biochips to be produced, use is made of compositions different in makeups (Table). An illustration is afforded by methods of producing the biochips using a composition K86.

A mixture of 2-methacrylamido-2-desoxy-D-glucose (29, 85 x 10⁻³ g, 9, 46 x 10⁻⁵ mole) and N, N'-methylenebisacrylamide (0.15 x 10⁻³ g, 0.97 x 10⁻⁶ mole) is added more with a solution of N,N,N',N'-tetramethylethylenediamine in deionized water (370 mcl, 13.5%), mixing to the complete dissolving of dry components. Glycerol is then poured in (500 mcl) as is an oligonucleatide solution in water (100 mcl, C=2000 pmole/mcl). The mixture is carefully mixed to form a homogeneous solution. The composition thus obtained is transferred to polymer substrates using a robot QArray (England, the firm "Genetix") in the form of an array of drops. The substrates with drop arrays are irradiated with UV light (λ=312 nm, 45 min., T=40°C) in a dry argon atmosphere.

Biochips are washed out at first in a phosphate buffer (0.005 M, pH 7.0) comprising Tween 20 (0.1 %) and sodium chloride (0.15 M) and in water then.

After drying in air, the biochips are ready-to-use.

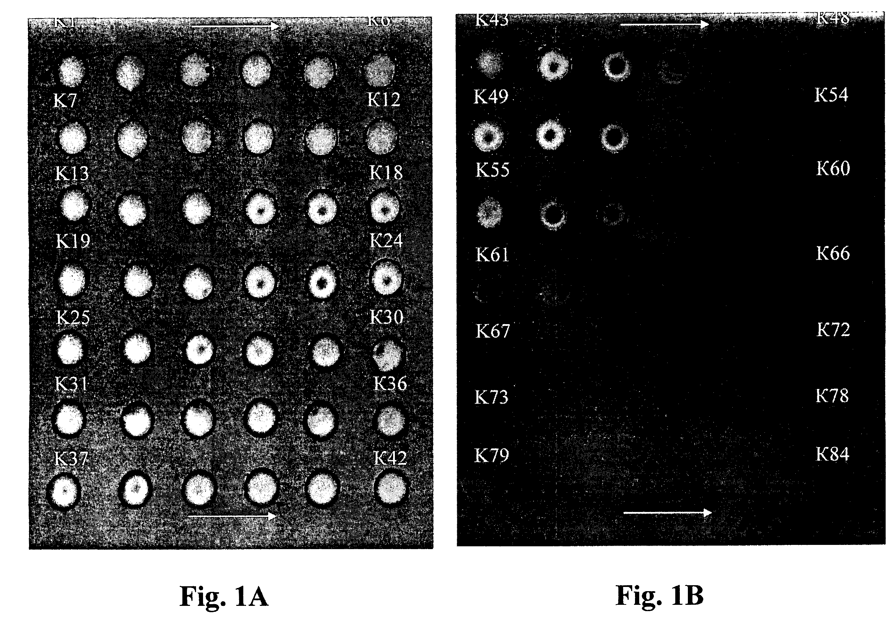

The biochips thus obtained were photographed in visible transmitted light. As seen from Fig. 1, the use of N-substituted aminosugar-based compositions allows one to produce hydrogels (A) in which 0 < **M** +**C** ≤ 3% (when 0 ≤**C**/(**C+M**) ≤ 50%) and 0 < **M** +**C** ≤ 0% (when 0 ≤**C**/(**C+M**) ≤ 3%). Whereas the use of a literature-known monomer - methacrylamide for producing the hydrogels of the same formulation does not provide the desirable result (B).

### Method 2. Production of protein biochips

To produce biochips, use is made of compositions having different makeups (Table). By way of example, methods of producing biochips are given using a composition K88.

To a mixture of 2-methacrylamido-2-desoxy -D-glucose (29.25 x - 10⁻³ g, 9.27 x 10⁻⁵ mole), N,N'-methylenebisacrylamide (0.525 x 10⁻³ g, 3.395 x 10⁻⁶ mole) and 2-acryloxoethylmethacrylate (0.225 x 10⁻³ g, 1.223 x 10⁻⁶ mole) added more is a solution of N,N,N',N'-tetramethylethylenediamine in deionized water (370 mcl, 3.24%), mixing until dry components are dissolved completely. Glycerol (500 mcl) is then poured in as is a protein solution in waer (100 mcl, C = 1 mg/ml). The mixture is carefully stirred to obtain a homogeneous solution.

The compositrion so obtained is transferred in the form of drops onto glass substrates treated with 3-trimethoxysilylpropylmethacrylate with the aid of a robot QArray (England, the firm "Genetix").

Drop array substrates are irradiated with UV light having a wave length of 350 nm and an intensity of 0.06 mcW/cm² (GTE lamps F1T8/350 BL, Sylvania, Danvers, MA) for 50 min. at 22°C. Prior to an immunoassay, biochips are washed off in a buffer solution (phosphate-saline buffer 0.01 M containing 0.15 M NaCl, ph 7.2 and 0.1% Tween 20) and then with water. After drying in air, the biochips are ready-to-use.

### Example 3. Hybridization performed on oligonucleotide biochips

The 5'-terminal Cy5 stain fluorescent labeled single-stranded DNA fragment of a 126 nucleotides rpoB M.tuberculosis gene that is obtained by a method of double-stage PCR (Mikhailovich V. et al., J.Clin. Microbiol., 2001, 39(7): 2531-40), was hybridized on an oligonucleotide biochip produced by using compositions K85 and K86 and oligonucleotides: 5'-TTC TGG TCC ATG AAT TGG-OCH₂CH(CH₂OH)(CH₂)₄-NH₂(A), 5'-GTT CTG GTC TAT GAA TTG G-OCH₂CH(CH₂OH)(CH₂)₄-NH₂(**B**) (Example 2 (method 1)). Hybridization was performed using a buffer (1.5 M guanidinethiocyanate, 0.075 M HEPES (pH 7.5), 7.5 M EDTA) at 37°C for three hours. Fluorescent signals from biochip cells were registered directly in a hybridization solution chamber (Fig. 2) using a fluorescent PZS chamber - provided microscope.

As can really be seen from the drawing cited, even for a small-in-size DNA fragment (126 n.o.) biochips based on one of the K86 claimed hydrogels are twice, as to a hybridization signal, the literature-known hydrogel with a minimum content of monomers and a minimum content of a cross-linker--K85 (patent RU 2216547). Also, discriminant relations between complete and incomplete duplexes do not change appreciably.

### Example 4. Performance of multiplex allel-specific PCR on biochip gel elements

Asymmetric multiplex PCR with M. tuberculosis DNA was performed in biochip gel elements in accordance with data of the work thus far published (Op. cit.). For the production of biochips, use was made of compositions K86 and K87 (cf. Table I). The regions of three parts of genes were amplified with the help of three pairs of primers: IS6110 (M. tuberculosis specific gene); katG (a gene encoding a catalase involved in isoniaside metabolism); rpoB (a gene of a sub-unit of a RNA polymerase, wherein the mutations necessitate stability to the isoniaside). Lengths of the amplified regions were 210 b.p. , 130 b.p. and 170 b.p., respectively.

Straight primers comprising wild type sequences or having at the 3'-terminal a single nucleotide substitution characterizing a mutant variant were immobilized in biochip cells. Reverse primers comprised a fluorescent label at the 5'-terminal and were in a reaction mixture.

For PCR to be performed, use was made of a buffer (2.5 mM MgCl₂, 10 mM KCI, 10 mM Tris-HCl (pH 8.3), 1 mg/ml BSA, 0.2 mM of each dNTR) with the addition of DNA-polymerase 5U Stoffel-Taq fragment (PE Corporation). The matrix used is represented by 10⁵ replicas of genomic *Mycobacterium tuberculosis* DNA. Thermocyclization parameters: initial warming up - 2 min. at 95°C, further - 35 cycles for 30 s, at 95°C; 60 s, at 63°C; 40 s, at 72°C. The results were registered upon completion of PCR with the help of a fluorescent PZS chamber-provided microscope.

The largest fluorescent signal was observed in cells, wherein the immobilized primer was completed to a stable duplex with a fluorescent labeled single-stranded DNA fragment of each and every gene that was produced during PCR (Fig. 3). Besides, it can be seen from the drawing that the value of a fluorescent signal on hydrogels obtainable from a composition K86 exceeds that on the hydrogels of a composition K87 by four odd times, which fact testifies to a greater efficiency of PCR.

### Example 5. Direct analysis of oncomarkers and kinetic measurements on biochip gel elements

For a direct analysis of oncomarkers and kinetic measurements to carried out, there have been produced biochips whose gel elements were produced on compositions K88-K91 basis in which the anti-prostate-specific antigen (PSA), serological anti-cancer prostate gland oncomarker monoclonal antibodies were immobilized (Example 2 (method 2)). For the reproducibility of results to be controlled, four equal gel elements each formed on a biochip for each composition of a hydrogel comprising the immobilized anti--bodies in equal concentrations.

A direct analysis and kinetic measurements were carried out in a phosphate-saline buffer (C=0,01 M, 0.15 M NaCl, pH 7.2) containing a 0.15% polyvinyl alcohol, a 0.15% and 100 ng/ml oncomarker. The fluorescent signals of gel elements were registered directly in a hybridization solution chamber, using a fluorescent PZS- chamber-provided microscope. Kinetic measurements were carried out at 15 min. intervals for 20 hrs, at 20°C.

As seen from the drawing (Fig.4), a maximum fluorescent signal is achieved using a composition K88, a minimum signal was received from K91 gel cells. The efficacy of immobilization of the antibodies in all gels was almost equal to constitute 35-40%.

All patent documents, publications, scientific articles and other documents and materials cited or mentioned here are included in the present specification by referring to such an extent if each and every document of interest was included by way of reference individually or cited here in its full form.

Despite the fact that the present invention was described in greater detail with a reference made to concrete alternative embodiments, a person skilled in the art will apparently grasp an idea of various alterations and modifications to be likely made without departing from a concept and an amount of the present invention which are defined by the claims attached.

**Table. Composition makeups usable for producing biochips**

| **No composition** | Composition components | | | | | | **Content and ratios of components, %** | | | | | |
|---|---|---|---|---|---|---|---|---|---|---|---|---|
| | **M** | **C** | B | S | Init | Inh | **M+C** | **C/** (M+C) | **B** | **S** | **Init** | Inh |
| K1 | 2-methacryl-amido-2-desoxy-D-glucose (MAADG) | N,N'-methylene- bisacryl-amide (MBAA) | no | Glycerol/ water 50/37 | N,N,N',N'-Tetramethy-lethylene-diamine (TMEDA) | no | 8 | 5 | 0 | Glycerol/ water 87 | N,N,N',N'-Tetramethy -lethylene-diamine 5 | 0 |
| K2 | MAADG | MBAA | no | 50/37 | TMEDA | no | 8 | 4 | 0 | 87 | 5 | 0 |
| K3 | MAADG | MBAA | no | 50/37 | TMEDA | no | 8 | 3 | 0 | 87 | 5 | 0 |
| K4 | MAADG | MBAA | no | 50/37 | TMEDA | no | 8 | 2 | 0 | 87 | 5 | 0 |
| K5 | MAADG | MBAA | no | 50/37 | TMEDA | no | 8 | 1,5 | 0 | 87 | 5 | 0 |
| K6 | MAADG | MBAA | no | 50/37 | TMEDA | no | 8 | 1 | 0 | 87 | 5 | 0 |
| K7 | MAADG | MBAA | no | 50/39 | TMEDA | no | 6 | 5 | 0 | 89 | 5 | 0 |
| K8 | MAADG | MBAA | no | 50/39 | TMEDA | no | 6 | 4 | 0 | 89 | 5 | 0 |
| K9 | MAADG | MBAA | no | 50/39 | TMEDA | no | 6 | 3 | 0 | 89 | 5 | 0 |
| K10 | MAADG | MBAA | no | 50/39 | TMEDA | no | 6 | 2 | 0 | 89 | 5 | 0 |
| K11 | MAADG | MBAA | no | 50/39 | TMEDA | no | 6 | 1,5 | 0 | 89 | 5 | 0 |
| K12 | MAADG | MBAA | no | 50/39 | TMEDA | no | 6 | 1 | 0 | 89 | 5 | 0 |
| K13 | MAADG | MBAA | no | 50/40 | TMEDA | no | 5 | 5 | 0 | 90 | 5 | 0 |
| K14 | MAADG | MBAA | no | 50/40 | TMEDA | no | 5 | 4 | 0 | 90 | 5 | 0 |
| K15 | MAADG | MBAA | no | 50/40 | TMEDA | no | 5 | 3 | 0 | 90 | 5 | 0 |
| K16 | MAADG | MBAA | no | 50/40 | TMEDA | no | 5 | 2 | 0 | 90 | 5 | 0 |
| K17 | MAADG | MBAA | no | 50/40 | TMEDA | no | 5 | 1,5 | 0 | 90 | 5 | 0 |
| K18 | MAADG | MBAA | no | 50/40 | TMEDA | no | 5 | 1 | 0 | 90 | 5 | 0 |
| K19 | MAADG | MBAA | no | 50/41 | TMEDA | no | 4 | 5 | 0 | 91 | 5 | 0 |
| K20 | MAADG | MBAA | no | 50/41 | TMEDA | no | 4 | 4 | 0 | 91 | 5 | 0 |
| K21 | MAADG | MBAA | no | 50/41 | TMEDA | no | 4 | 3 | 0 | 91 | 5 | 0 |
| K22 | MAADG | MBAA | no | 50/41 | TMEDA | no | 4 | 2 | 0 | 91 | 5 | 0 |
| K23 | MAADG | MBAA | no | 50/41 | TMEDA | no | 4 | 1,5 | 0 | 91 | 5 | 0 |
| K24 | MAADG | MBAA | no | 50/41 | TMEDA | no | 4 | 1 | 0 | 91 | 5 | 0 |
| K25 | MAADG | MBAA | no | 50/42 | TMEDA | no | 3 | 5 | 0 | 92 | 5 | 0 |
| K26 | MAADG | MBAA | no | 50/42 | TMEDA | no | 3 | 4 | 0 | 92 | 5 | 0 |
| K27 | MAADG | MBAA | no | 50/42 | TMEDA | no | 3 | 3 | 0 | 92 | 5 | 0 |
| K28 | MAADG | MBAA | no | 50/42 | TMEDA | no | 3 | 2 | 0 | 92 | 5 | 0 |
| K29 | MAADG | MBAA | no | 50/42 | TMEDA | no | 3 | 1,5 | 0 | 92 | 5 | 0 |
| K30 | MAADG | MBAA | no | 50/42 | TMEDA | no | 3 | 1 | 0 | 92 | 5 | 0 |
| K31 | MAADG | MBAA | no | 50/43 | TMEDA | no | 2 | 5 | 0 | 93 | 5 | 0 |
| K32 | MAADG | MBAA | no | 50/43 | TMEDA | no | 2 | 4 | 0 | 93 | 5 | 0 |
| K33 | MAADG | MBAA | no | 50/43 | TMEDA | no | 2 | 3 | 0 | 93 | 5 | 0 |
| K34 | MAADG | MBAA | no | 50/43 | TMEDA | no | 2 | 2 | 0 | 93 | 5 | 0 |
| K35 | MAADG | MBAA | no | 50/43 | TMEDA | no | 2 | 1,5 | 0 | 93 | 5 | 0 |
| K36 | MAADG | MBAA | no | 50/43 | TMEDA | no | 2 | 1 | 0 | 93 | 5 | 0 |
| K37 | MAADG | MBAA | no | 50/44 | TMEDA | no | 1 | 5 | 0 | 94 | 5 | 0 |
| K38 | MAADG | MBAA | no | 50/44 | TMEDA | no | 1 | 4 | 0 | 94 | 5 | 0 |
| K39 | MAADG | MBAA | no | 50/44 | TMEDA | no | 1 | 3 | 0 | 94 | 5 | 0 |
| K40 | MAADG | MBAA | no | 50/44 | TMEDA | no | 1 | 2 | 0 | 94 | 5 | 0 |
| K41 | MAADG | MBAA | no | 50/44 | TMEDA | no | 1 | 1,5 | 0 | 94 | 5 | 0 |
| K42 | MAADG | MBAA | no | 50/44 | TMEDA | no | 1 | 1 | 0 | 94 | 5 | 0 |
| K43 | Methacryl-amide (MAA) | MBAA | no | 50/37 | TMEDA | no | 8 | 5 | 0 | 87 | 5 | 0 |
| K44 | MAA | MBAA | no | 50/37 | TMEDA | no | 8 | 4 | 0 | 87 | 5 | 0 |
| K45 | MAA | MBAA | no | 50/37 | TMEDA | no | 8 | 3 | 0 | 87 | 5 | 0 |
| K46 | MAA | MBAA | no | 50/37 | TMEDA | no | 8 | 2 | 0 | 87 | 5 | 0 |
| K47 | MAA | MBAA | no | 50/37 | TMEDA | no | 8 | 1,5 | 0 | 87 | 5 | 0 |
| K48 | MAA | MBAA | no | 50/37 | TMEDA | no | 8 | 1 | 0 | 87 | 5 | 0 |
| K49 | MAA | MBAA | no | 50/39 | TMEDA | no | 6 | 5 | 0 | 89 | 5 | 0 |
| K50 | MAA | MBAA | no | 50/39 | TMEDA | no | 6 | 4 | 0 | 89 | 5 | 0 |
| K51 | MAA | MBAA | no | 50/39 | TMEDA | no | 6 | 3 | 0 | 89 | 5 | 0 |
| K52 | MAA | MBAA | no | 50/39 | TMEDA | no | 6 | 2 | 0 | 89 | 5 | 0 |
| K53 | MAA | MBAA | no | 50/39 | TMEDA | no | 6 | 1,5 | 0 | 89 | 5 | 0 |
| K54 | MAA | MBAA | no | 50/39 | TMEDA | no | 6 | 1 | 0 | 89 | 5 | 0 |
| K55 | MAA | MBAA | no | 50/40 | TMEDA | no | 5 | 5 | 0 | 90 | 5 | 0 |
| K56 | MAA | MBAA | no | 50/40 | TMEDA | no | 5 | 4 | 0 | 90 | 5 | 0 |
| K57 | MAA | MBAA | no | 50/40 | TMEDA | no | 5 | 3 | 0 | 90 | 5 | 0 |
| K58 | MAA | MBAA | no | 50/40 | TMEDA | no | 5 | 2 | 0 | 90 | 5 | 0 |
| K59 | MAA | MBAA | no | 50/40 | TMEDA | no | 5 | 1,5 | 0 | 90 | 5 | 0 |
| K60 | MAA | MBAA | no | 50/40 | TMEDA | no | 5 | 1 | 0 | 90 | 5 | 0 |
| K61 | MAA | MBAA | no | 50/41 | TMEDA | no | 4 | 5 | 0 | 91 | 5 | 0 |
| K62 | MAA | MBAA | no | 50/41 | TMEDA | no | 4 | 4 | 0 | 91 | 5 | 0 |
| K63 | MAA | MBAA | no | 50/41 | TMEDA | no | 4 | 3 | 0 | 91 | 5 | 0 |
| K64 | MAA | MBAA | no | 50/41 | TMEDA | no | 4 | 2 | 0 | 91 | 5 | 0 |
| K65 | MAA | MBAA | no | 50/41 | TMEDA | no | 4 | 1,5 | 0 | 91 | 5 | 0 |
| K66 | MAA | MBAA | no | 50/41 | TMEDA | no | 4 | 1 | 0 | 91 | 5 | 0 |
| K67 | MAA | MBAA | no | 50/42 | TMEDA | no | 3 | 5 | 0 | 92 | 5 | 0 |
| K68 | MAA | MBAA | no | 50/42 | TMEDA | no | 3 | 4 | 0 | 92 | 5 | 0 |
| K69 | MAA | MBAA | no | 50/42 | TMEDA | no | 3 | 3 | 0 | 92 | 5 | 0 |
| K70 | MAA | MBAA | no | 50/42 | TMEDA | no | 3 | 2 | 0 | 92 | 5 | 0 |
| K71 | MAA | MBAA | no | 50/42 | TMEDA | no | 3 | 1,5 | 0 | 92 | 5 | 0 |
| K72 | MAA | MBAA | no | 50/42 | TMEDA | no | 3 | 1 | 0 | 92 | 5 | 0 |
| K73 | MAA | MBAA | no | 50/43 | TMEDA | no | 2 | 5 | 0 | 93 | 5 | 0 |
| K74 | MAA | MBAA | no | 50/43 | TMEDA | no | 2 | 4 | 0 | 93 | 5 | 0 |
| K75 | MAA | MBAA | no | 50/43 | TMEDA | no | 2 | 3 | 0 | 93 | 5 | 0 |
| K76 | MAA | MBAA | no | 50/43 | TMEDA | no | 2 | 2 | 0 | 93 | 5 | 0 |
| K77 | MAA | MBAA | no | 50/43 | TMEDA | no | 2 | 1,5 | 0 | 93 | 5 | 0 |
| K78 | MAA | MBAA | no | 50/43 | TMEDA | no | 2 | 1 | 0 | 93 | 5 | 0 |
| K79 | MAA | MBAA | no | 50/44 | TMEDA | no | 1 | 5 | 0 | 94 | 5 | 0 |
| K80 | MAA | MBAA | no | 50/44 | TMEDA | no | 1 | 4 | 0 | 94 | 5 | 0 |
| K81 | MAA | MBAA | no | 50/44 | TMEDA | no | 1 | 3 | 0 | 94 | 5 | 0 |
| K82 | MAA | MBAA | no | 50/44 | TMEDA | no | 1 | 2 | 0 | 94 | 5 | 0 |
| K83 | MAA | MBAA | no | 50/44 | TMEDA | no | 1 | 1,5 | 0 | 94 | 5 | 0 |
| K84 | MAA | MBAA | no | 50/44 | TMEDA | no | 1 | 1 | 0 | 94 | 5 | 0 |
| K85 | MAA | MBAA | Oligo-nucle-atide | 50/ 41,999 989 | TMEDA | no | 3 | 3 | 0,00 0011 | 91,9 9998 9 | 5 | 0 |
| K86 | MAADG | MBAA | Oligo-nucle-atide | 50/ 41,999 989 | TMEDA | no | 3 | 0,5 | 0,00 0011 | 91,9 9998 9 | 5 | 0 |
| K87 | MAA | MBAA | Oligo-nucle-atide | 50/ 41,999 989 | TMEDA | no | 3 | 3 | 0,00 0018 | 91,9 9998 9 | 5 | 0 |
| K88 | MAADG | MBAA+ 2-acryl-oxoethyl-methacry-late (AM) | protein | 50/ 45,79 | TMEDA | no | 3 | 2,5 | 0,01 | 95,7 9 | 1,2 | 0 |
| K89 | MAADG | MBAA+ AM | protein | 50/44, 79 | TMEDA | no | 4 | 2,5 | 0,01 | 94,7 9 | 1,2 | 0 |
| K90 | MAADG | MBAA+ AM | protein | 50/ 43,79 | TMEDA | no | 5 | 4 | 0,01 | 93,7 9 | 1,2 | 0 |
| K91 | MAADG | MBAA+ AM | protein | 50/ 42,79 | TMEDA | no | 6 | 5 | 0,01 | 92,7 9 | 1,2 | 0 |

## Claims

1. A monomer for producing low percentage hydrogel and/or hydrogel having a low "cross-linkage" content representing an O-substituted sugar and/or N-substituted aminosugar-based derivative comprising at a hydroxyl or amino group, respectively, an unsaturated residue.

2. The monomer according to claim 1, **characterized in that** a sugar is a low molecular compound of a class of carbohydrates, selected from the group consisting of monosaccharides and oligosaccharides.

3. The monomer according to claim 2, **characterized in that** the sugar is a compound selected from the group consisting of saccharose, D-glucose, D-allose, D-altrose, D-mannose, D-gulose, D-idose, D-talose, D-fructose, D-ribose, D-arabinose, D-xylose, D-lyxose, D-eritrulose, D-ribulose, D-xylulose, D-psicose, D-sorbose, D-magatose or their desoxyanalogue or their analogue with the L-configuration or a mixture of isomers with L and D-configurations.

4. The monomer according to claim 1, **characterized in that** an amino sugar is a natural or synthetic sugar in which one or several hydroxyl groups are substituted with an amino group.

5. The monomer according to claim 4, **characterized in that** an amino sugar is a compound selected from the group consisting of 2-amino-2-desoxy-D-glucose, 2-amino-2-desoxy-D-allose, 2-amino-2-desoxy-D-altrose, 2-amino-2-desoxy-D-mannose, 2-amino-2-desoxy-D-gulose, 2-amino-2-desoxy-D-idose, 2-amino-2-desoxy-D-talose, 2-amino-2-desoxy-D-galactose or analogues thereof with the L-configuration or a mixture of isomers with D and L-configurations.

6. The monomer according to claim 1, **characterized in that** the unsaturated residue employed is represented by a group containing a multiple bond being caused to react with photo or chemical initiated polymerization.

7. The monomer according to claim 6, **characterized in that** the unsaturated residue employed is represented by a methacryl, acryl, vinyl, allyl, 4-vinylbenzoyl or methylene-4-phenylenevinyl group.

8. A composition (C) for producing low-percentage hydrogel and/or hydrogel having a low "cross-linkage" content, comprising:
M - monomer according to any one of claims 1-7, forming a base of the formable gel;
C - cross-linker;
S - solvent.

9. The composition according to claim 8, **characterized in that** the monomer (M) further used is represented by at least one other unsaturated monomer.

10. The composition according to claim 9, **characterized in that** the at least said one other unsaturated monomer used is represented by a compound selected from the group comprising methacrylamide, acrylamide, 2-hydroxyethylmethacrylate and 2-hydroxyethylmethacrylamide.

11. The composition according to claim 8, **characterized in that** the cross-linker (C) used is represented by at least one unsaturated compound comprising two or more multiple bonds.

12. The composition according to claim 11, **characterized in that** the at least said one unsaturated compound used is represented by a compound selected from the group comprising N,N'-methylenebisacrylamide, N,N'-(1,2-dihydroxyethylene)bisacrylamide, polyethylene glycol diacrylate.

13. The composition according to claim 8, **characterized in that** the solvent(S) represents a solution of organic and/or inorganic compound.

14. The composition according to claim 13, **characterized in that** the solvent is selected from the group consisting of an aqueous solution of glycerol, sugar, betaine, polyalcohol; a solution of glycerol, sugar, betaine or polyalcohol in dimethylsulfoxide (DMSO); a solution of glycerol, sugar, betaine or polyalcohol in dimethylformamide (DMFA).

15. The composition according to claim 8, **characterized in** further comprising at least one initiator and/or polymerization promoter (Init).

16. The composition according to claim 15, **characterized in that** the initiator and/or promoter of polymerization is an initiator for chemical initiated polymerization and/or promoter for photoinitiated polymerization.

17. The composition according to claim 16, **characterized in that** at least one initiator and/or promoter of polymerization is selected from the group consisting of ammonium persulfate, potassium persulfate, hydrogen peroxide, benzoyl peroxide, azoisobutyronitrile (AIBN), methylene blue, fluorescein, N,N,N',N'-tetramethylethylenediamine, 4- (N,N-dimethylamino)pyridine, triethylamine, and acetone.

18. The composition according to claim 8, **characterized in** further comprising at least one inhibitor (Inh),

19. The composition according to claim 18, **characterized in that** the least said one polymerization inhibitor (Inh) is a compound precluding spontaneous polymerization.

20. The composition according to claim 19, **characterized in that** the at least said one spontaneous polymerization precluding compound is a compound selected from the group consisting of n-benzoquinone, o-nitrophenol, p-nitrophenol, nitrobenzene, o-dinitrobenzene, m-dinitrobenzene and p - dinitrobenzene.

21. The composition according to claim 8, **characterized in** further comprising at least one fluorescent stain (St).

22. The composition according to claim 21, **characterized in that** the at least said one stain used is represented by the one selected from the group consisting of Texas Red, fluorescein, Cy 5, Cy 4, BODIPY.

23. The composition according to any one of claims 8-22, **characterized in** further comprising a biologically significant compound (B).

24. The composition according to claim 23, **characterized in that** the biologically significant compound (B) is capable of being immobilized in a hydrogel at the moment of its formation.

25. The composition according to claim 23 or 24, **characterized in that** the biologically significant compound contained in the composition is represented by at least one compound selected from the group consisting of a nucleotide, a nucleic acid, a protein, an oligosaccharide, an antibody or a low-molecular ligand.

26. A hydrogel representing a low-percentage hydrogel and/or hydrogel having a low "cross-linkage" content, produced by the photo-or chemical induced polymerization of a composition according to any one of claims 8-25.

27. A biochip representing a substrate with a hydrogel layer immobilized thereon according to claim 26.

28. The biochip according to claim 27, **characterized in that** the hydrogel layer is a continuous layer.

29. The biochip according to claim 27, **characterized in that** the hydrogel layer is divided by voids into cells.

30. The biochip according to claim 29, **characterized in that** the cells form a regular unidimensional or two-dimensional structure (array).

31. The biochip according to claim 27, **characterized in that** the gel layer comprises at least one immobilized biologically significant compound.

32. The biochip according to claim 31, **characterized in that** the immobilization of at least one biologically significant compound in the hydrogel layer is performed at the moment of gel formation in chemical- or photochemical initiated polymerization.

33. The biochip according to claim 31, **characterized in that** the immobilization of at least one biologically significant compound in the hydrogel layer is performed after the hydrogel has been formed.

34. The biochip according to any one of claims 29-33, **characterized in that** each and every biochip hydrogel cell comprises an immobilized fluorescent stain.


SEQUENCE LISTING

<110>  Uchrezhdenie Rossiiskoi Akademii Nauk Institut
       Molekulyarnoi Biologii IM. V.A. Engelgardta Ran (IMB RAN)

<120>  Monomer and composition for producing low-percentage hydrogel
       and/or hydrogel having low cross linkage content hydrogel and
       biochip based thereon

<130>  137967

<140>  PCT/RU2007/000196
<141>  2007-04-20

<160>  2

<170>  PatentIn version 3.4

<210>  1
<211>  18
<212>  DNA
<213>  Artificial

<220>
<223>  oligonucleotide


<220>
<221>  misc_feature
<222>  (18)..(18)
<223>  OCH2CH(CH2OH)(CH2)4-NH2 at the 3' end of the oligonucleotide

<400>  1
ttctggtcca tgaattgg                                                    18


<210>  2
<211>  19
<212>  DNA
<213>  Artificial

<220>
<223>  oligonucleotide


<220>
<221>  misc_feature
<222>  (19)..(19)
<223>  OCH2CH(CH2OH)(CH2)4-NH2 at the 3' end of the oligonucleotide

<400>  2
gttctggtct atgaattgg                                                   19